# EUROPEAN PATENT APPLICATION

(11) **EP 3 248 608 A1**
(43) Date of publication of application: **29.11.2017**
(21) Application number: 17179262.5
(22) Date of filing: 18.02.2014
(51) Int. Cl.: A61K 36/88, A61K 31/55, A61P 29/00, C07D 491/056

(54) **PROCESS FOR EXTRACTING THE ALKALOID FRACTION OF RHODOPHIALA BIFIDA (HERB.) TRAUB AND USES THEREOF**

(30) Priority: 18.02.2013 BR 102013003718
(62) Divisional of application: 14751108.3
(71) Applicant: Universidade Federal Do Rio Grande Do Sul-UFRGS, CEP 90040-060, Porto Alegre, RS (BR); Hospital De Clínicas De Porto Alegre (HCPA), 90035-903 Porto Alegre - RS (BR)
(72) Inventor: GNIESLAW DE OLIVEIRA,, Patricia, 90690-130 Porto Alegre, RS (BR); PEREIRA RAMOS PEDRAZZA,, Graziele, 91530-010 Porto Alegre, RS (BR); FARINON,, Mirian, 90050-260 Porto Alegre, RS (BR); RICARDO MACHADO,, Xavier, 91530-008 Porto Alegre, RS (BR); SILVEIRA ZUANAZZI,, José Angelo, 91530-008 Porto Alegre, RS (BR); SPIES,, Fernanda, 91610-000 Porto Alegre, RS (BR)
(74) Representative: Potter Clarkson LLP

(57) **Abstract**

The present invention provides an alkaloid fraction of *Bhodophiala Bifida* (Herb.) Traub for use in the treatment or prevention of inflammation, rheumatoid arthritis, ulcerative colitis, sepsis, acute pulmonary disease, inflammatory infections, such as inflammatory and/or fibrotic lung and/or kidney diseases, osteoporosis, Castleman's disease, psoriatic arthritis, juvenile rheumatoid arthritis or further non-specific inflammatory joint diseases.

## Description

### Field of the invention

The present invention discloses a process for the complete extraction of the alkaloid fraction (montanine) from bulbs of *Rhodophiala bifida* (Herb.) Traub. The present invention further describes a method for treating or preventing diseases involving in its pathogenesis the exacerbated migration or proliferation of lymphocytes and/or fibroblasts, with the ability to modify the course of the disease without changing or depressing the immune system when systemically administered using pharmaceutical compositions containing the alkaloid fraction of *Rhodophiala bifida* as an active ingredient and/or montanine alone or in admixture.

The present invention thus comprises an extraction process of the alkaloid fraction of *Rhodophiala bifida,* which is faster than other processes described in the literature, not requiring several cycles of solvent exchange in order to deplete the plant and the use of the alkaloid fraction as an active ingredient that is able to act in a preventive or curative manner: (i) controlling fibroblast migration and/or proliferation; (ii) controlling lymphocyte migration and/or proliferation; (iii) modifying inflammatory diseases, especially inflammatory arthritis; and (iv) being administered systemically without changing the patient's immune system. The use of the present invention is characterized by the development of a medicament for anti-inflammatory use for the treatment and/or prevention of diseases having inflammation and/or the localized increase in the number of fibroblasts as etiopathogenesis, namely: Rheumatoid Arthritis, ulcerative colitis, sepsis, acute lung disease, particularly inflammatory and fibrotic diseases related the lungs and kidneys, osteoporosis, Castleman's disease, psoriatic arthritis, juvenile rheumatoid arthritis, and other non-specific inflammatory joint diseases. Said drug can also be administered systemically, modifying the course of the disease, without changing or depressing the patient's immune system.

### State of the art

Plants from the Amaryllidaceae family are used in African and European countries in traditional medicine as emetic, purgative and antiparasitic agents. Alkaloids isolated from the bulbs of these plants have relevant pharmacological activities, such as antiviral activity, anti-inflammatory activity and anticholinergic activity - the best representative of the latter is galantamine, a drug used in the treatment of Alzheimer's disease, which is the active fraction of Razadyne^{®}, formerly known as Reminyl, a drug approved by the FDA in 2001, and the cytotoxic anti-tumor activity of licorine, which is another relevant alkaloid from this family.

Recent articles published in 2013 demonstrate the presence of the alkaloid montanine in other genera of the Amaryllidaceae family As an example, we can mention: *Haemanthus* L, *Scadoxus, Cryptostephanus, Hippeastrum, Rhodophiala;* species where montanine can be found: *Haemanthus albiflos* Jacq, *Haemanthus defonis* Hook f., *Haemanthus hirsutus* Baker, *Haemanthus coccineus* L, *Haemanthus sanguineus* Jacq, *Haemanthus montanus* Baker, *Scadoxus puniceus* (L.) Friis & Nordal, *Cryptostephanus vansonii* Verd, *Hippeastrum vittatum* (L'Her.) Herbert, *Rhodophiala bifida* (Herb.) Traub.

Recently, there has been a great scientific advance involving chemical and pharmacological studies of medicinal plants for the purposes of obtaining new compounds having therapeutic properties. This is because plants have contributed over the years to the provision of various drugs, which even today, are widely used in the clinic, namely: morphine, emetine, vincristine, galantamine, among others.

In this context, the number of plants on the planet should be highlighted, wherein most of them is unknown from a scientific point of view, where from among between 250 and 500 thousand species only about 5% of them were studied in terms of their chemical profiles and a smaller percentage was evaluated considering biological aspects.

Within the broad plant diversity, the Amaryllidaceae family has shown to be very promising due to a number of very characteristic and unique alkaloids. From among the Amaryilidaceae alkaloids, galantamine has to be mentioned, which inhibits the acetylcholinesterase enzyme and has already been introduced in the therapy against Alzheimer's disease through the Razadyne^{®} drug, former Reminyl, which was approved by the FDA in 2001. This large monocot family comprises 59 genera and 870 species. One of these genera is *Hippeastrum,* which is endemic in America (from Argentina to Mexico) and is described in only a few studies in the literature and some species have never been studied.

Literature data demonstrate that the biological activity and toxic effects of Amaryilidaceae family plants are caused by the presence of alkaloids. These compounds isolated from Amaryilidaceae species have shown to exhibit a great pharmacological potential and several studies have reported the interest in this class of substances for cancer therapy, as antivirals, antimalarials and analgesics in addition to having activity on the CNS, which has galantamine as main representative.

*Rhodophiala bifida* (Herb.) Traub is a native species from northeast Argentina, also found in Uruguay and Brazil. It was first identified by Herbert in 1837, belongs to the Hippeastreae tribe, Amaryilidaceae family, Lillifloreae order, Monocotyledoneae class. The plant is characterized by flowering in the end of the summer (during the month of March), having a black, subspherical bulb having a diameter in the range of from 3 to 4 cm and fleshy, linear, leaves of up to 30 cm in length and about 1 cm in width, usually after the flowering. It has an umbel inflorescence (3 to 4) with 2 to 7 flowers having unequal pedicels, a perianth of 4 to 5 cm and purple petals. The stamens are uneven having white, pink and declined filaments. The anthers have length ranging from 5 to 6 mm and stigma is trefoiled (tri-lobed). The *Rhodophiala* genus is very close taxonomically to the Hippeastrum genus. Older studies have classified the *Rhodophiala* gender as *Hippeastrum.*

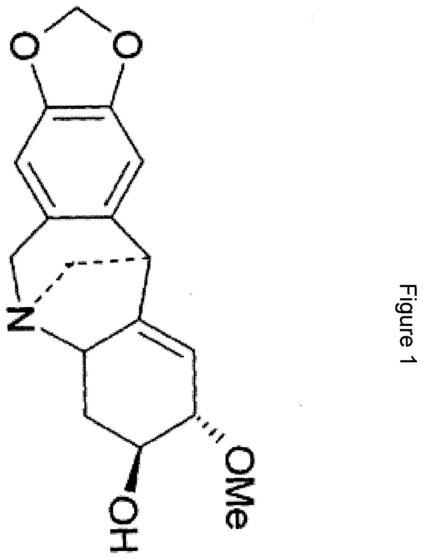

Montanine - Figure 1 - is an isoquinolinic alkaloid extracted and isolated from the bulbs of *Rhodophiala bifida* (Herb.) Traub. Results demonstrating that montanine exhibited psychopharmacological activities, including anxiolytic, antidepressant and anticonvulsant effects have been published recently. However, to date, the efficacy of montanine as active compound capable of acting on inflammation, migration and proliferation of fibroblasts and lymphocytes, inflammatory disease modification; and also able to be administered systemically without modifying or depressing the immune system were unknown.

Inflammation is the organism's reaction to infection, ischemia, toxic agents, autoimmunity, or tissue injury, which is characterized by the reaction of blood vessels, leading to fluid and leukocyte accumulation in order to destroy, dilute and isolate damaging agents or the immune system itself. Leukocytes, in turn, destroy the damaged tissue and send signals to macrophages which ingest and digest antigens and dead tissue. In some diseases, said process may exhibit a destructive character and treatment will depend on the cause of inflammation. The process usually leads to the cure of the infection and tissue repair.

Initially, inflammation is said to be acute when there are changes in the caliper and blood flow, increased permeability and leukocyte migration. Its cardinal signs are pain, heat, redness and swelling. Pain is the main symptom of acute inflammation as it causes a major limitation of daily activities. Furthermore, there is also a signaling and communication process through the production of pro-inflammatory proteins such as cytokines and chemokines produced by immune system cells. However, as acute inflammation becomes uncontrollable by the regulatory mechanisms of the immune system it is said to be a chronic inflammation, which is usually caused by the permanence of the aggressor. At this point the major cellular components involved in the process are macrophages and soluble components. Fibroblasts are deemed the main components in the transformation of acute to chronic inflammation, which ultimately causes deformity and loss of function of the affected joint. Because of the production of cytokines by the initial acute inflammation, synovial fibroblasts migrate into the affected joint. Once inside the joint, they build up and cause a mechanical effect of joint thickening, causing deformity and pain. In addition, synovial fibroblasts produce several cytokines, which act as chemical signaling molecules, including interleukin-6, which stimulates T lymphocytes to migrate into the joint, leading to the maintenance of the intra-articular inflammatory condition. Another important action of fibroblasts is its ability to transform into myofibroblasts and participate of the fibrosis production, causing irreparable joint damage. Finally, both synovial fibroblasts and T lymphocytes can secrete RankL, which promotes differentiation and activation of osteoclasts, causing bone erosion adjacent to the joint. Since chronic inflammation does not cease until the control mechanisms are restored or until the "trigger" for inflammation development is withdrawn from the body, such process can take days, months or even years. Also, a body of evidence shows that diseases such as cancer and coronary heart diseases may be closely related to chronic inflammatory processes.

Current anti-inflammatory treatments are divided into two major groups:
I. The ones that are said to be hormonal (steroids), also known as glucocorticoids, *corticoids* or corticosteroids, are agents that inhibit the production of prostaglandins and leukotrienes by inhibiting phospholipase A enzyme, via the release of lipocortin-1 (an anti-inflammatory protein mediator). Glucocorticoids reduce the transcription of several inflammatory proteins, such as certain cytokines, induced nitric oxide synthase and cyclooxygenase 2. Such effect explains most of their pharmacological actions. However, corticoids are only symptomatic, not being able to slow the progression of chronic inflammatory diseases such as rheumatoid arthritis. In addition, the use thereof has several dose- and time-dependent drawbacks, including weight gain, diabetes mellitus, hypertension and immunosuppression, the latter causing an increased risk for infection.
II. The non-hormonal ones promote inhibition of cyclooxygenase, interfering in the production of prostaglandins, prostacyclins and thromboxanes. They have reduced action over the former ones, as they will not inhibit leukotrienes since lipoxygenase remains active, thus maintaining part of the inflammatory process unchanged. Its main use is in the reduction of the symptoms of inflammation, such as pain and edema. Some examples are *aspirin* and *ibuprofen.* Like corticosteroids, they are only symptomatic, not being able to modify the natural course of chronic arthritis.

A second grouping can be applied to currently known treatments: (i) those that act upon the reduction or control of symptoms; and (ii) those capable of modifying the disease. In particular, the treatment of rheumatoid arthritis uses a class of drugs designated as *Disease-Modifying Antirheumatic Drug* (DMARD), which has the ability to prevent the disease from progressing (from inflammation to joint remodeling) and not only to treat their symptoms. Despite its high efficacy, DMARDs are expensive (in rule, due to its origin, as some of them are biologics), they are administered systemically as an injection and promote depression of the patient's immune system, which is the more undesired side effect this class of drugs.

In general, the anti-inflammatory drugs currently used to treat acute or chronic inflammation also have significant side effects, including allergic reactions; gastropathy (esophagitis, gastric bleeding, etc.); nephropathy (interstitial nephritis, kidney failure, among other disabilities); in the heart they can lead to heart failure and increased risk for acute myocardial infarction; hepatotoxicity. As a consequence, current drugs having anti-inflammatory activity still have high adverse effects and toxicity, and for this reason the search for therapies that affect the body less aggressively becomes so important.

Based on montanine activity, the following diseases can be prevented, treated or controlled:
I: acute and chronic inflammatory diseases, including rheumatoid arthritis and juvenile rheumatoid arthritis, diseases in which the fibroblast acts as the main mechanism for transforming acute into chronic inflammation; psoriatic arthritis, arthropathy associated with psoriasis characterized by irreversible deformity and joint destruction, the main disease triggering and perpetuation mechanism of which are T lymphocytes; as montanine causes reduction of pain and clinical scores when administered preventatively or for the treatment of active diseases, it can be used as symptomatic for any diseases involving joint inflammation, including osteoarthritis or arthrosis.
II: Fibrotic diseases: including lung and renal fibrotic diseases, since it inhibits the migration of fibroblasts and the consequent transformation thereof into myofibroblasts, resulting in fibrosis.
III: Castleman's disease: benign lymphoproliferative disease characterized by an increase in hyperplastic lymph nodes, for which the only treatment currently available is chemotherapy and radiation. The main fisiopathological mechanism is increased interleukin-6, a cytokine that is inhibited by montanine.
IV: Osteoporosis: a disease that weakens the bones increasing the risk for fractures, whose main mechanism of action is the excessive production of RankL, a factor produced by fibroblasts and T lymphocytes.

The use of medicinal plants in its various forms has grown in this century. From the main drug therapy used in the first decades, it has declined to such an extent that it has almost become extinct. Nowadays, it occupies again a key role in primary health care, a fact which is supported by the WHO guidelines, consolidated in the document "Estratégia de la OMS sobre Medicina Tradicional 2002-2005" in the final report of the "1^{a} Conferência Nacional de Medicamentos e Assistência Farmacêutica (1st National Conference on Drugs and Pharmaceutical Assistance)" held in Brasilia on September 2003 as well as the guidelines of the current National Policies for Natural Medicine and Complementary Practices developed by the Ministry of Health. According to the current policy for the regulation of medicines in Brazil, as published by Anvisa in 2004, Phytotherapy understands that plant extracts, compounds from naturally produced substances, are as safe and effective, or even safer and more effective, than those produced synthetically.

The alkaloid fraction of *Rhodophiala bifida* bulbs (montanine) has been poorly studied so far. To date, only psychopharmacological activities, including anxiolytic, antidepressant and anticonvulsant effects, and a large antimicrobial potential were reported. Regardless of these few findings with significant effects, the molecule appears to be promising for testing for other purposes.

It is important to note that the search for new therapeutic strategies for inflammatory diseases is a strategic and evident necessity to optimize management of these diseases. Therefore, we hereby intend to present results showing the role of montanine.

Four relevant documents have been found in the state of the art:
EP2001877A1, *"Method for extracting target alkaloid using an ionic liquid as extracting solvent,* which describes a method for extracting a target alkaloid from a mixture of species, usually from a plant material, using an ionic liquid as an extraction solvent. The ionic liquid may be an alkanolyl, alkoxyalkyl- or aminoalkyl-substituted ammonium salt. Said patent differs from our proposal as it uses different solvents to extract the alkaloid and a different isolation process. Patent US 7968734, *"Organocatalysts and methods of use in Chemical synthesis"* concerns reactions usually comprising organocatalysts that facilitate stereo-selective reactions as well as the method for the synthesis use thereof. This patent describes a reaction for the synthesis of montanine, which is different from our proposal, which is to extract montanine from a plant.

The document "Anti-inflamatory activity of alkaloids; a twenty-century review", Revista Brasileira de Farmacognosia, 16 (1): 109-139, Jan/Mar - 2006, presents on page 120, reference to *Tabernae montanine* as an active anti-inflammatory agent. Despite the similarity in the designation, the described molecule is noticeably distinct from that covered in the patent;

In the document "Avaliação in vitro das atividades anti-inflamatórias, antioxidantes e antimicrobianas do alcalóide montanina" Revista Brasileira de Farmacognosia, 17 (2): Abr/Jun - 2007 the molecule described in the patent had its anti-inflammatory effect challenged (at the concentration of 100 micrograms/mL) and was unable to generate a different effect than the placebo on the lymphocyte migration model. Therefore, the publication does not render the anti-inflammatory effect of the patented molecule obvious; rather it points to a different direction;

No prior art documents were found to destroy the novelty requirement of the present invention.

### BRIEF DESCRIPTION OF THE INVENTION

The present invention details an extraction process of the alkaloid fraction of *Rhodophiala bifida,* which is faster than other processes described in the literature and does not require numerous exchanges of solvents in order to lead to depletion of the plant. Isolation requires a single step, ensuring high yield and purity of alkaloid.

Still little studied, the montanine alkaloid exhibits psychopharmacological activities including anxiolytic, antidepressant and anticonvulsant effects and a large antimicrobial potential, showing its potential as a novel therapeutic strategy for many purposes. However, the use thereof as an anti-inflammatory agent has not been described so far. Similarly, the ability thereof to inhibit migration and proliferation of fibroblasts and lymphocytes has not been described. The understanding that montanine is able to modify inflammatory disease without changing the patient's immune system is also a novel object presented herein. Thus, the present patent application relates to the extraction of this alkaloid and its potential as: an anti-inflammatory agent; a drug candidate capable of reducing pain and the clinical scores of acute and chronic arthritis both prophylactically and for treatment of joint diseases; a drug candidate capable of controlling migration and proliferation of fibroblasts and lymphocytes; a drug candidate capable of modifying inflammatory disease without changing or depressing the immune system with particular application in rheumatoid arthritis, but considering the use thereof in other diseases whose pathogenesis involves local inflammation and/or migration and/or ectopic proliferation of fibroblasts.

It should be noted that it is important to search for new therapeutic strategies for inflammatory and fibrotic diseases and it becomes evident in view of the need for strategies that provide for an optimized management of these diseases.

It is an object of the present invention a method for extracting and isolating the alkaloid fraction of *Rhodophiala bifida* from bulbs of *Rhodophiala bifida.*

In a preferred aspect the method comprises the following steps:
a. Firstly the bulbs of *Rhodophiala bifida* are washed with tap water, cut into chips and dried in a stove;
b. after drying, the bulbs are milled in a knife mill;
c. subjecting the dried and milled bulbs to a liquid medium with sulfuric acid at a concentration of 0.5 to 50%, at the ratio of 0.5 to 5 grams of bulbs to 5 to 50 mL of the acidic solution and are placed in an ultra-sound bath for a time that ranges from 1 to 48 hours at a temperature ranging from ambient to 100 °C. Preferably the following conditions are used: sulfuric acid 2% (or hydrochloric acid 50%) at the ratio of 1g of bulb to 10 mL of acidic solution, for 4 hours in the ultra-sound bath at room temperature;
d. centrifuging the mixture and basifying the supernatant;
e. extracting the basified solution with apolar solvents, preferably ethyl acetate (or ethyl ether, petroleum ether, chloroform, benzene, Dichloromethane), which is thereafter evaporated;
f.subjecting the residue of the organic phase to vacuum chromatography utilizing hexane as mobile phase and silica as the stationary phase;
g. performing isolation of montanine using a type of polar solvent, preferably C1 to C4 alcohols, preferably using methanol;
h. evaporating methanol; the dry residue is then frozen to be freeze-dried;
i. freeze-drying to obtain an alkaloid fraction of *Rhodophiala bifida.*

It is a further object of the present invention the use of montanine, as described in Figure 1, as an active ingredient to prevent, treat and/or control acute or chronic inflammations, based on the following lymphocyte migration and proliferation tests.

It is a further object of the present invention the use of montanine, as described in Figure 1, as an active ingredient capable of preventing invasion or migration of fibroblasts, acting on the treatment and/or control of diseases involving this element in its etiopathogenesis, based on the following fibroblast migration/invasion tests.

It is a further object of the present invention the use of montanine, as described in figure 1, as an active ingredient capable of being applied systemically without causing effects or depressing the patient's immune system based on the following immunosuppression tests.

It is a further object of the present invention the use of montanine, as described in Figure 1, as an active ingredient capable of modifying inflammatory disease, with special emphasis on the inflammatory disease of the osteoarticular system and for rheumatoid arthritis, without causing the undesired side effect of immune system depression based the lymphocyte and fibroblast migration and proliferation tests as well as the following immunosuppression tests.

Certain aspects of the invention are summarized in the following numbered paragraphs:
1. PROCESS FOR EXTRACTING THE ALKALOID FRACTION OF *RHODOPHIALA BIFIDA* (HERB.) TRAUB AND USES THEREOF, **characterized** by being from bulbs of *Rhodophiala bifida,* but not limited to it, being extendable to other genera and species from the Amaryllidaceae family which also contain montanine: genera *Haemanthus* L, *Scadoxus, Cryptostephanus, Hippeastrum, Rhodophiala or Haemanthus albiflos* Jacq, *Haemanthus defonis* Hook f., *Haemanthus hirsutus* Baker, *Haemanthus coccineus* L, *Haemanthus sanguineus* Jacq, *Haemanthus montanus* Baker, *Scadoxus puniceus* (L) Friis & Nordal, *Cryptostephanus vansonii* Verd, *Hippeastrum vittatum* (L'Hér.) Herbert, *Rhodophiala bifida* (Herb.) Traub *species.*
2. PROCESS FOR EXTRACTING THE ALKALOID FRACTION OF *RHODOPHIALA BIFIDA* (HERB.) TRAUB AND USES THEREOF of paragraph 1, **characterized** by comprising the following steps:
   a. cleaning the *Rhodophiala bifida* bulbs with tap water, cutting them into chips and drying in a stove;
   b. grinding the bulbs in a knife mill after drying;
   c. subjecting the dried and grinded bulbs to an acidified liquid medium, placing in an ultra-sound bath, heating;
   d. centrifuging the mixture and basifying the supernatant;
   e. extracting the basified solution with organic solvents;
   f.subjecting the residue of the organic phase to vacuum chromatography utilizing hexane as mobile phase;
   g. Isolating montanine using a polar solvent, preferably, C1 to C4 alcohols;
   h. evaporating the solvent and the dry residue is then frozen for further freeze-drying;
   i. freeze-drying to obtain montanine.
3. THE ALKALOID FRACTION OF *RHODOPHIALA BIFIDA* (HERB.) TRAUB AND USES THEREOF, **characterized** by being for the treatment or prevention of inflammation, rheumatoid arthritis, ulcerative colitis, sepsis, acute pulmonary disease, inflammatory infections and particularly inflammatory and fibrotic lung and kidney diseases, osteoporosis, Castleman's disease, psoriatic arthritis, juvenile rheumatoid arthritis and further non-specific inflammatory joint diseases.
**4**. The use of montanine either isolated or present in any pharmaceutically acceptable composition, **characterized** by being for the treatment or prevention of diseases having migration - invasion or ectopic proliferation of fibroblasts as etiopathogenesis, such as rheumatoid arthritis and juvenile rheumatoid arthritis; also, as an active ingredient used in the treatment or prevention of diseases whose etiopathogenesis derives from the inflammatory process, lymphocyte migration and/or proliferation, such as non-specific acute and chronic arthritis, psoriatic arthritis and Castleman's disease; also, that is for preventing or treating inflammatory and/or fibrotic diseases of the osteo-articular system, particularly osteoporosis, fibrotic lung forms of rheumatoid arthritis, idiopathic pulmonary fibrosis and renal and retroperitoneal fibrosis; in addition, the use of montanine having the ability of modifying the course of disease without causing an impact or depressing the immune system.

These and other objects of the invention will be immediately appreciated by those skilled in the art and by interested companies in the field and will be described in sufficient detail for reproduction in the following description.

### BRIEF DESCRIPTION OF DRAWINGS

Figure 1 shows the chemical structure of the alkaloid montanine
Figure 2 demonstrates the chromatogram with mass spectrometry detection of isolated montanine
Figure 3 demonstrates the chromatogram with detection in the UV region of isolated montanine
Figure 4 shows the mass spectrum of montanine
Figure 5 shows the effect of montanine on leukocyte migration to the femorotibial articulation and joint hypernociception in a model of mBSA-induced monoarthritis. *P < 0.05
Figure 5A demonstrates leukocyte migration
Figure 5B shows hypernociception
Figure 6 shows the effect of montanine on lymphocyte viability for 24h. *P < 0.05
Figure 7 shows the effect of montanine on the LPS- or ConA-induced lymphocyte proliferation. *P < 0.05
Figure 7A shows the LPS-induced lymphoproliferation
Figure 7B shows the ConA-induced lymphoproliferation
Figure 8 shows the effect of montanine on synovial fibroblast invasion. *P < 0.05
Figure 9 shows the cell blood count, immunosuppression test of montanine, at time zero, after 3 days of administration and after 3 more days with no administration given.
Figure 10 shows the cell viability in 24 hours with different concentrations of montanine.
Figure 11A shows lymphoproliferation with lipopolysaccharide (LPS).
Figure 11B shows lymphoproliferation with conconavalin A (ConA).
Figure 12A shows the invasion of fibroblasts with and without 1 µM montanine.
Figure 12B shows the invasion of fibroblasts with and without 1 µM montanine.

### Detailed description of the invention

The general methods for the extraction of alkaloids are based on solubility in water-immiscible organic solvents (ether, ethyl acetate, benzene, etc.) and water insolubility. Alkaloid salts exhibit inverse properties.

In the present invention there is described an effective method for the extraction of the alkaloid fraction of *Rhodophiala bifida.*

The bulbs of *Rhodophiala bifida* are first washed with tap water, cut into chips and dried in a stove until complete removal of water. Thereafter, the dry bulbs are milled in a knife mill.

For extraction of chemical substances, subjecting the dried and milled bulbs to a liquid medium with sulfuric acid at a concentration of 0.5 to 50%, at a ratio of 0.5 to 5 grams of bulbs to 5 to 50 mL of the acidic solution and are placed in an ultra-sound bath for a time that ranges from 1 to 48 hours at a temperature ranging from ambient to 100 °C. Preferably the following conditions are used: sulfuric acid 2% (or hydrochloric acid 50%) at the ratio of 1g of bulb to 10 mL of acidic solution for 4 hours in the ultra-sound bath at room temperature;

Thereafter, the mixture is centrifuged and the supernatant is basified. The basified solution is extracted with ethyl acetate (or ethyl ether, petroleum ether, chloroform, benzene, dichloromethane). Preferably, from among the nonpolar solvents ethyl acetate is used, which is then evaporated.

The residue of the organic phase is subjected to vacuum liquid chromatography, first using hexane as the mobile phase to remove possible impurities, and then the step of montanine isolation with solvents is performed using a polar solvent, preferably C1 to C4 alcohols, more preferably using methanol. To each 10 g of bulbs used in the extraction 100 mL of mobile phases are used, respectively. Methanol is then evaporated and the dry residue is frozen to be freeze-dried. After freeze-drying the yield of alkaloid fraction is of approximately 4% relative to the dry bulbs.

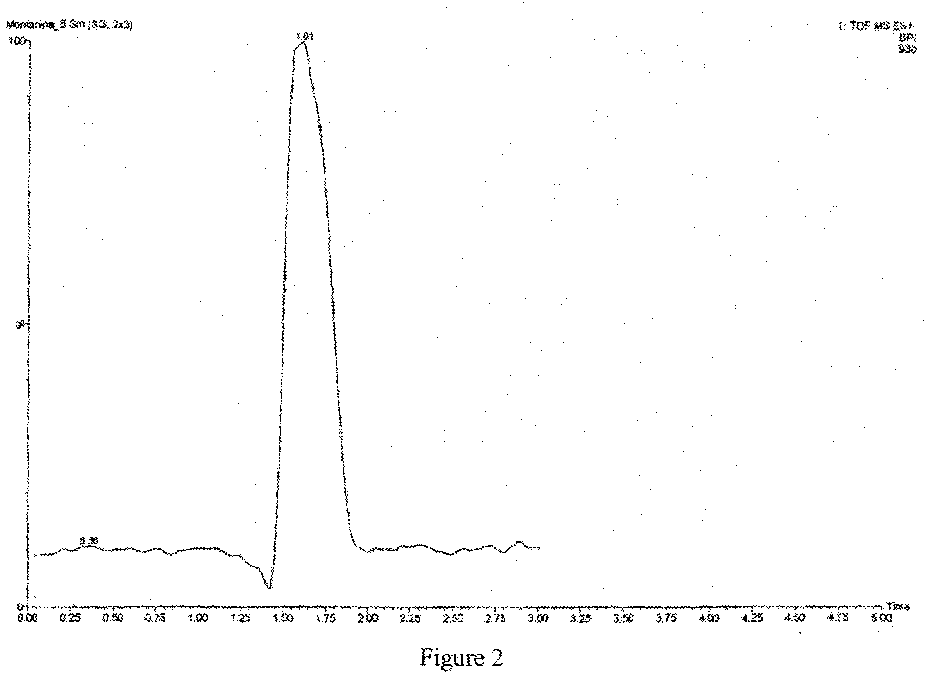

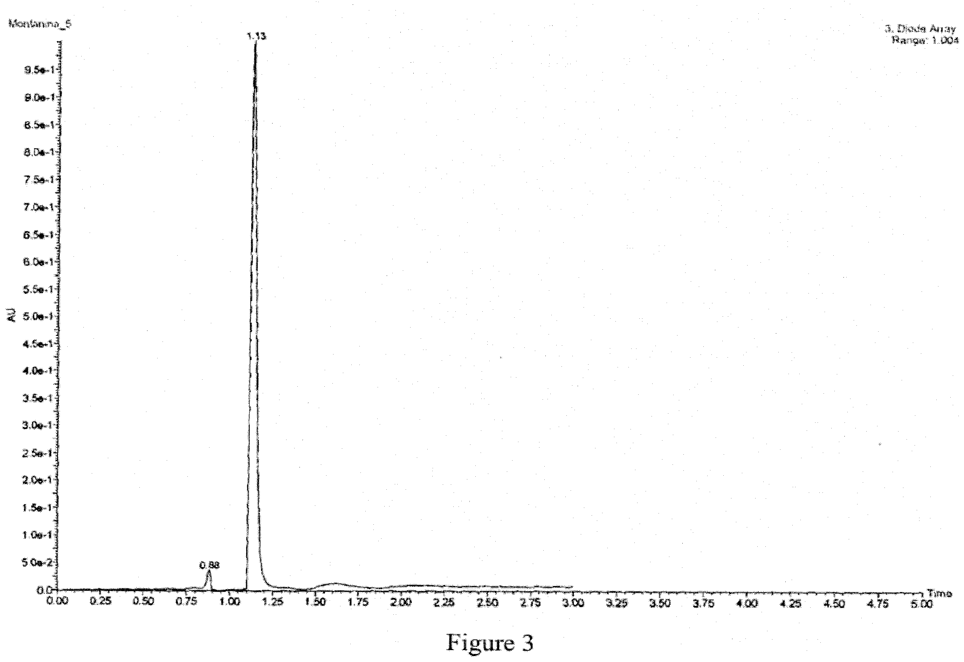

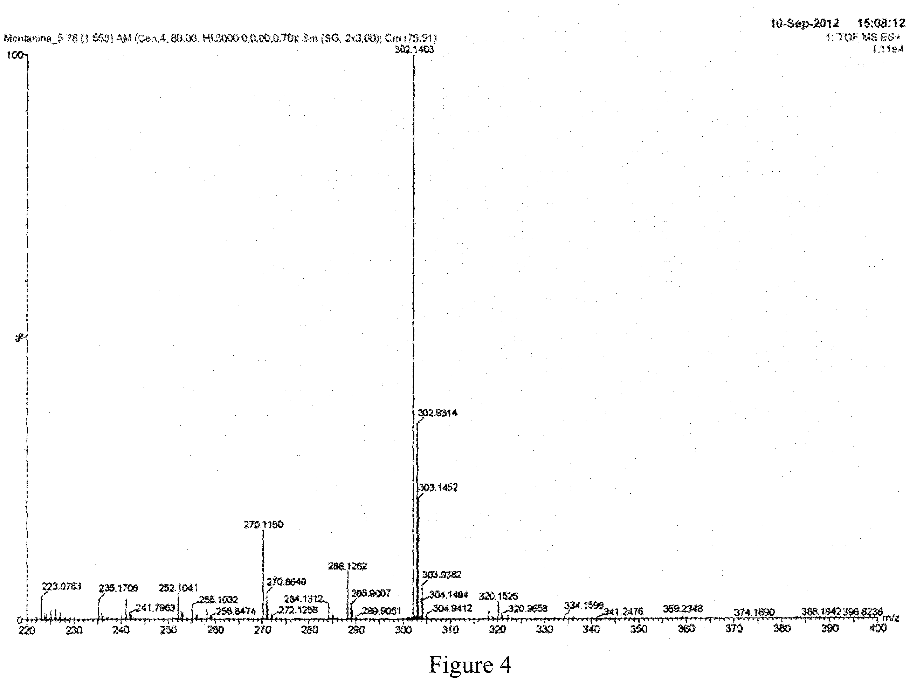

To examine the identity of the isolated fraction High-Performance Liquid Chromatography with ultraviolet (UV) and/or mass spectrometry (MS) detection is used. In Figures 2 and 3 we disclose the MS and UV chromatograms of the isolated fraction, respectively.. As can be noted in Figure 3, the alkaloid fraction comprises a single substance, which is shown in the peak at 1.13 minutes. In Figure 4 we depict the mass spectrum of the isolated substance where the values of calculated mass = 301,1314 Da; calculated mass (+1) = 302.1392 Da; confirm the experimental mass = 302.1403 Da of the alkaloid montanine, confirming the product's identity. Difference = 3.64 ppm, below 5 ppm of exact mass demonstrates the identity of montanine.

To solubilize montanine in 0.9% physiological saline, the solution was placed in an ultra-sound bath for 2 hours for it to be used as a treatment.

### Description of the experiments

The present invention further proposes the montanine activity, as described in figure 1, to be an anti-inflammatory, such as an inhibitor of lymphocyte migration, such as an inhibitor of fibroblast migration/invasion and inhibitor of T-lymphocyte proliferation; as a modifier of inflammatory disease, especially that related to the osteoarticular system, with emphasis on rheumatoid arthritis without causing changes or depression of the patient's immune system when administered systemically.

Below we describe experiments that led to the conclusion that montanine is effective as an anti-inflammatory agent.

### - In vivo experiments

### Antigen-induced arthritis (AIA) model

Antigen-induced arthritis (AIA) is an animal model of arthritis that is broadly described and used in worldwide literature. One of the inducing antigens in this model in Balb-C mice is methylated bovine serum albumin (mBSA), which, after systemic immunization (subcutaneous injection) is injected into the joint. AIA is an immune-mediated (T cell-dependent) joint inflammation, whose histopathology shows many similarities with rheumatoid arthritis in humans (Grespan et al. 2008).

Briefly, mice were immunized in three steps with mBSA (day-0, day-7 and day-14), the first step comprising 500 mg of mBSA protein in an emulsion of 0.1 mL of complete Freund's adjuvant's (CFA) and 0.1 mL of sterile saline solution (0.9% sodium chloride), the second and third steps were performed with the same protein concentration, the same sterile saline solution concentration and the same adjuvant concentration, but in these steps incomplete Freund's adjuvant (IFA) was used; 3 weeks after the first immunization (day 21) mBSA at a concentration of 30 ug/mL were injected into the femorotibial joint (knee) and the contralateral knee was administered with sterile saline only and serves as the control of the experiment (Grespan et al. 2008). The injected joint developed an acute inflammation within a few hours (characterized by massive granulocyte infiltration and fibrin exudate) and from the behavioral point of view, mice showed pronounced mechanical hyperalgesia (pain) in the inflamed knee. Treatment with montanine was given twice daily, one day before intra-articular challenge, at the day of the challenge and at day of death, by intraperitoneal route and diluted in saline. Doses tested were 0.3; 1; 3 mg/kg. One group of animals received the treatment vehicle (saline) for control purposes, said group being designated positive control. The experiment is terminated at day 22 (24 hours after challenge and treatment) when animals were euthanized to collect the joint lavage fluid. Lavage was performed using a PBS-EDTA solution and then the number of leukocytes existing in the joint was counted in a Neubauer chamber at a 1:2 dilution with Turk's solution. Furthermore, pain of the animals was assessed in a digital analgesia-meter (von Frey) prior to intra-articular injection at time 0 (zero), 3, 5 and 24 hours after intra-articular challenge.

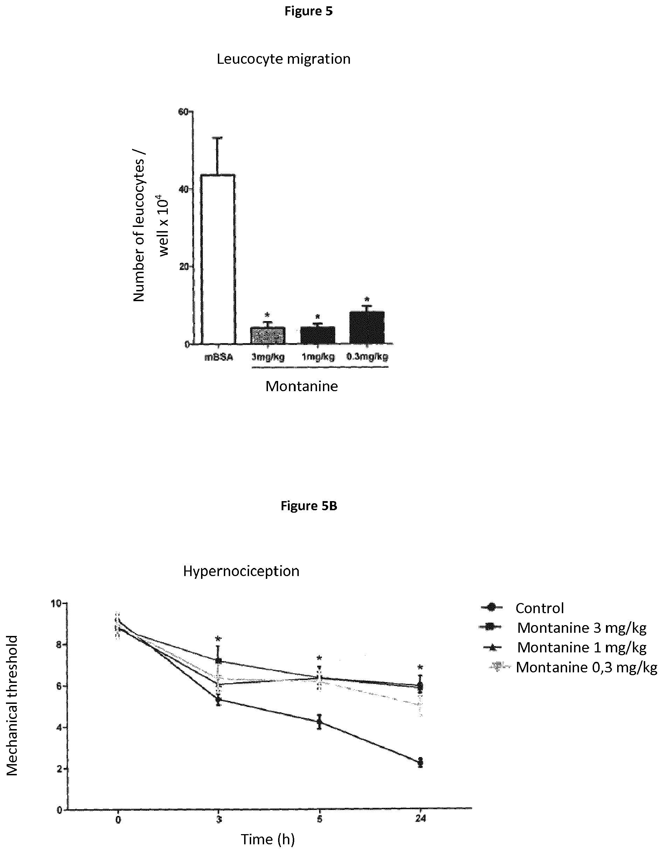

Montanine exhibited reduction in pain at all tested doses (Figure 5), but the dose of 3 mg/kg showed a decrease as of 3 hours. In addition, the total leukocyte migration was significantly reduced at all three doses (Figure 5) compared with the group receiving saline solution only.

### Collagen-induced arthritis (CIA) model with Prophylactic treatment

The chronic arthritis model most widely used in the world literature is the model of collagen type II-induced arthritis (CIA). Said model shares many pathological features with the disease, such as type II collagen (CM), the major cartilage protein and one of the potential RA self-antigens.

CIA has been widely used to identify the potential pathogenic mechanisms of autoimmunity, including the role of different cell types, individually, at the beginning and during the progression of the disease, as well as to test and develop new therapies.

Briefly, CIA was induced in DBA/1J mice (8-12 weeks, average weight of 20 g) which were immunized by 50µL of an emulsion containing equal volumes of bovine type II collagen (2mg/ml) and complete Freund's adjuvant (CFA) by intradermal injection (i.d.) at the base of the tail on day-0 and on day-18 after this first immunization a boost was given with an emulsion of IFA and CU, injected below the first site of injection.

After the boost injection, treatment at dosages of 0.05; 0,25 and 0.5 mg/kg was started and continued for 15 days (twice daily, intraperitoneally) and animals were monitored daily for clinical signs of the disease.

The following techniques for assessment of chronic arthritis and the effect of montanine on this model were analyzed:

### Arthritis clinical score

Animals were monitored daily for analyzing the clinical signs of arthritis by means of the severity score as follows: 0- no signs of disease; 1 - mild erythema and edema; 2 - moderate erythema and edema; 3 - severe erythema and edema extending from the knee to the metatarsus; 4 - severe erythema and edema with loss of function. The total score is the average of the scores on the legs from the onset of the disease.

### Histological analysis

The tibio-tarsal joints of DBA/1 J animals were isolated and immersed in 10% buffered formalin for fixation for 24 hours. Then, tissues were decalcified in 10% trichloroacetic acid (TCA) for approximately 18 hours. These tissues were dehydrated and embedded in paraffin blocks. Six µm-thick cuts were arranged on a microscope slide. Slides were stained with hematoxylin and eosin staining technique for assessment of the following parameters: synovial inflammation: five high-power magnification fields - HMF was analyzed for the percentage of infiltrating mononuclear cells: 0- absent, 1-mild (1-10%), 2-moderate (11-50%), 3- severe (51-100%); synovial hyperplasia: 0-absent, 1-mild (5-10 cell layers), 2-moderate (11-20 layers), 3-severe (>20 layers);extent of pannus formation: 0- absent, 1- mild, 2- moderate, 3- severe; synovial fibrosis: 0-absent, 1-mild (1-10%), 2-moderate (11-50%), 3-severe (51-100%); synovial vascularization (angiogenesis): sum of the number of vessels in five HMF of synovial tissue; cartilage erosion: 0-absent, 1-mild (1-10%), 2-moderate (11-50%), 3-severe (51-100%); bone erosion: 0- absent, 1- minor erosion(s) observed only in HMF, 2- moderate erosion(s) observed at low magnification, 3- severe transcortical erosion(s) as previously described by Oliveira et al. 2011 and for assessment of cartilage degradation analysis was performed using the safranin-O staining technique. All the cuts were assessed under a microscope by two blinded observers, and the images captured by digital camera.

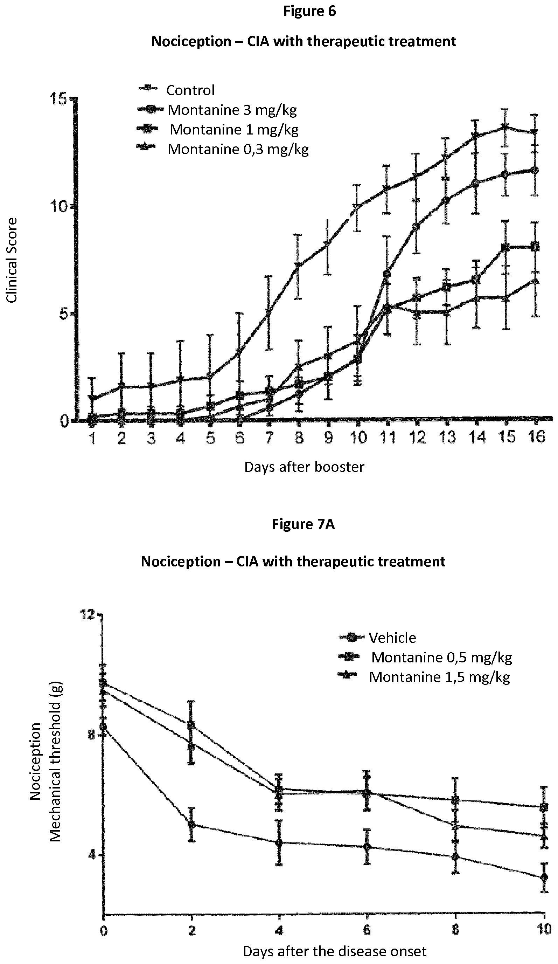

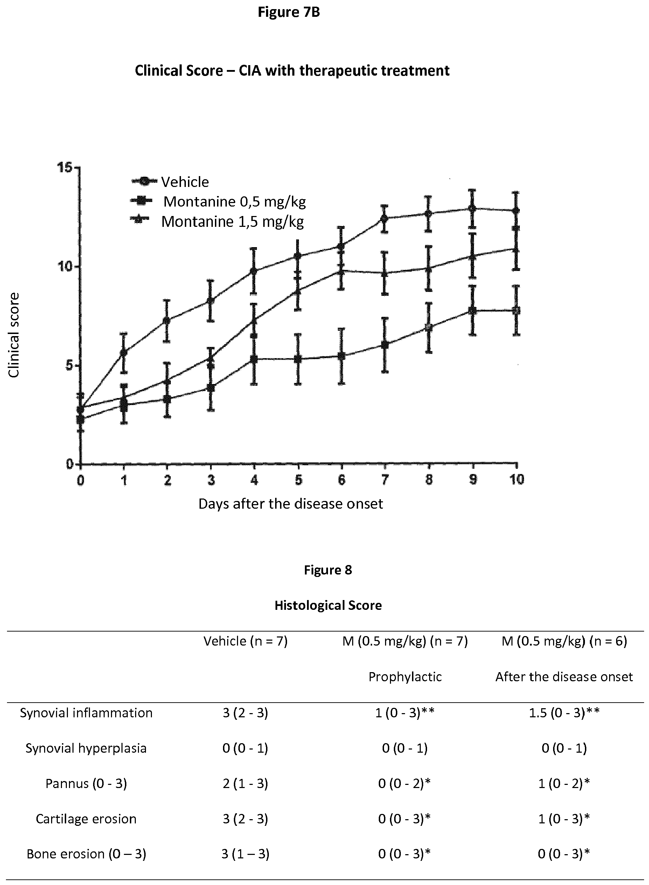

Prophylactic treatment with montanine exhibited reduced clinical score of the disease at doses of 0.25 and 0.5 mg/kg from day 8 of treatment (Figure 6). Furthermore, treatment at a dose of 0.5 mg/kg improved all the histological parameters of the joint, except for synovial hyperplasia (Figure 8).

### Collagen-induced arthritis (CIA) model with therapeutic treatment

CIA induction is performed by following the aforementioned protocol.

After the boost injection, animals were monitored daily for clinical signs of the disease and treatment with montanine at 0.5 and 1.5 mg/kg is started on the day of clinical detection of CIA. Animals given sterile saline, the treatment vehicle, were deemed as the positive control group, i.e. untreated. The drug was administered twice daily for 10 days by the intraperitoneal route.

The following techniques for assessment of chronic arthritis and the effect of montanine on this model were analyzed:

### Arthritis clinical score

Animals were monitored daily for analyzing the clinical signs of arthritis by means of the severity score as follows: 0- no signs of disease; 1 - mild erythema and edema; 2 - moderate erythema and edema; 3 - severe erythema and edema extending from the knee to the metatarsus; 4 - severe erythema and edema with loss of function. The total score is the average of the scores on the legs from the onset of the disease.

### Articular nociception

Articular hypernociception was assessed as previously described by Pinto LG *et al.* 2010. For this model, a polypropylene tip was adapted to the manual force transducer, a force was applied on the subplantar surface of the paw producing tibiotarsal bending motion. The automatic pressure meter recorded the strength of the applied force when the paw is removed. The assay is repeated 3 times sequentially to provide a consistent measurement, the limiar being expressed in grams (g).

### Histological analysis

The tibio-tarsal joints of DBA/1 J animals were isolated and immersed in 10% buffered formalin for fixation for 24 hours. Then, tissues were decalcified in 10% trichloroacetic acid (TCA) for approximately 18 hours. These tissues were dehydrated and embedded in paraffin blocks. Six µm-thick cuts were arranged on a microscope slide. Slides were stained with hematoxylin and eosin staining technique for assessment of the following parameters: synovial inflammation: five high-power magnification fields - HMF will be analyzed for the percentage of infiltrating mononuclear cells: 0- absent, 1-mild (1-10%), 2-moderate (11-50%), 3- severe (51-100%); synovial hyperplasia: 0-absent, 1-mild (5-10 cell layers), 2-moderate (11-20 layers), 3-severe (>20 layers); extent of pannus formation: 0- absent, 1- mild, 2- moderate, 3- severe; synovial fibrosis: 0-absent, 1-mild (1-10%), 2-moderate (11-50%), 3-severe (51-100%); synovial vascularization (angiogenesis): sum of the number of vessels in five HMF of synovial tissue; cartilage erosion: 0-absent, 1-mild (1-10%), 2-moderate (11-50%), 3-severe (51-100%); bone erosion: 0- absent, 1- minor erosion(s) observed only in HMF, 2- moderate erosion(s) observed at low magnification, 3- severe transcortical erosion(s) as previously described by Oliveira *et al.* 2011 and for assessment of cartilage degradation analysis the safranin-O staining technique was performed. All the cuts were assessed under a microscope by two blinded observers, and the images captured by digital camera.

Therapeutic treatment with montanine at the dosage of 0.5 mg/kg exhibited reduced pain (figure 7A), as well as a reduction of the clinical score from the third day of treatment (figure 7B). Furthermore, treatment at a dose of 0.5 mg/kg improved all the histological parameters, except for synovial hyperplasia (Figure 8).

### Immunosuppression assay

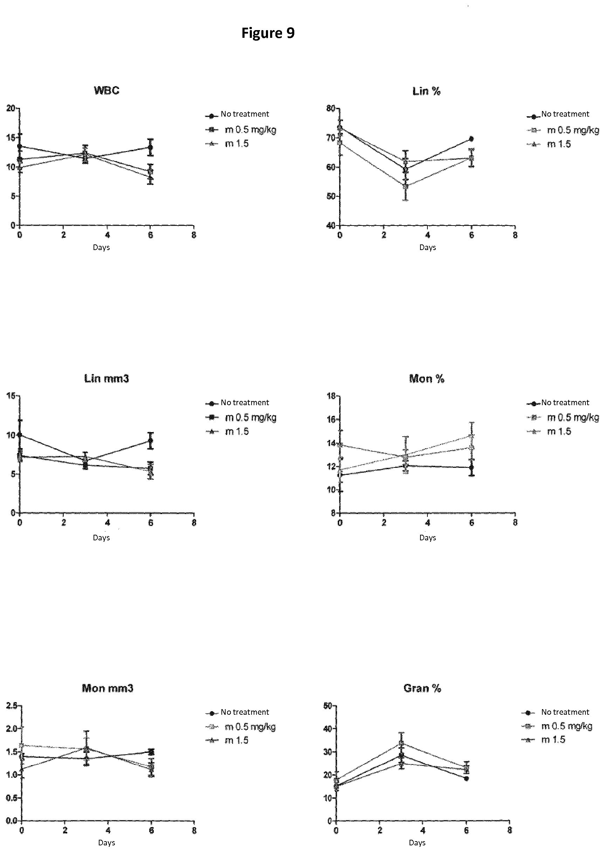

An immunosuppression assay in healthy animals was performed to assess the role of montanine as an immunosuppressive agent. 20 male, healthy mice of 8-12 weeks of age were used for examining the whole blood (complete blood count - parameters of: total of white blood cells (WBC); % lymphocytes (Lin%), lymphocytes/mm3 (Lin mm30; % monocytes (Mon%); monocytes/mm3 (Mon mm3); % granulocytes (Gran%); granulocytes/mm3 (Gran/mm3); total of red blood cells (RBC); hemoglobin (HGB) and total of platelets (PLQ)) of animals, which were divided as follows: healthy animals (with no treatment given) (n=4), animals treated with 0.5 or 1.5 mg/kg de montanine/ 12-12 hours (n=8/group). Whole blood was collected at day 0 for basal analysis of the blood parameters of the complete blood count in all animals, they were then treated for 3 days with the two doses and on the third day blood was collected again for complete blood count analysis; also, animals were still alive for three more days without any treatment for a new blood collection (figure 9).

This result has demonstrated that montanine does not lead to immunosuppression in any of the tested dosages as compared with the control e with each other.

### - In vitro experiments

### Lymphocyte viability test

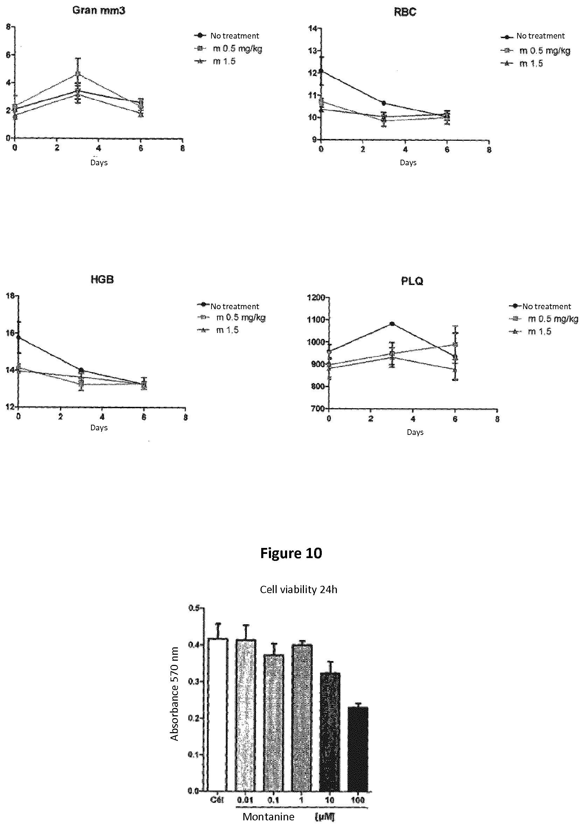

A lymphocyte viability test was performed to evaluate the cytotoxicity of montanine using a colorimetric MTT [3-(4,5-dimethylthiazol-2-yl) -2,5-diphenyl tetrazolium bromide] assay, as described by Tomita T. *et al.* 2006. Animals were euthanized and their draining lymph nodes (popliteal and inguinal) were removed aseptically. A single cell suspension was prepared, grown in triplicate (5 x 105 cells/well in a 96 well plate) and treated with montanine at the dosages (0.01 µM, 0.1 µM, 1 µM, 10µM and 100µM) for 48 hours at 37 °C at 5% CO₂ in RPMI medium. After incubation, MTT (0.5 mg/ml) was added to each well and the plate was placed in the stove again and after 4 hours the supernatant was removed and 50 µl of dimethyl sulphoxide (DMSO, Sigma) were added. After the plate was shaken to dissolve the MTT formazan crystals, optical density (OD) of each well was measured at 570 nm using a microplate ELISA reader. Montanine at doses of 0.01 µM, 0.1 µM and 1 µM did not alter cell viability (Figure 10). There is no significant difference between the cell group and the group treated with the montanine at 10 µM, however, decreased cell viability can be seen, which was higher in the 100 µM dose. From the results obtained in this assay, the dosage of 1 µM was chosen as the test dosage for subsequent in vitro assays, as this was the highest dose that did not exhibit any cytotoxic effects on the cells.

### Lymphocyte proliferation assay

The in vitro lymphocyte proliferation was performed using the MTT colorimetric assay described by Tomita T *et al.* 2006. BALB/c mice were euthanized and their draining lymph nodes were removed aseptically. A single cell suspension was prepared, grown in triplicate (5 x 105 cells/well in a 96 well plate) and treated with montanine at the dosages of 1 µM for 48h at 37 °C at 5% CO₂ in RPMI medium containing 10 mg/mL lipopolysaccharide (LPS) or 5 mg/mL of conconavalin A (ConA) or RPMI medium alone as a culture control. After incubation, MTT (0.5 mg/ml) was added to each well and the plate was placed in the stove again and after 4 hours the supernatant was removed and 50 µl of dimethyl sulphoxide (DMSO, Sigma) were added. The plate was placed in the stove again and the supernatant was then removed and 50 µl of DMSO (Sigma) were added. After the plate was shaken to dissolve the formazan crystals, optical density (OD) of each well was measured at 570 nm.

Both ConA and LPS are molecules that stimulate the lymphocyte proliferation, but they exhibit differences in terms of specificity. LPS acts primarily on the B cell receptor and the Toll-like receptor 4 (TLR4), molecules that are present on the surface of B lymphocytes, thus acting on these cells. ConA acts on several receptors containing glycoproteins or lipoproteins, stimulating both lymphocytes both but acting preferably on T lymphocytes.

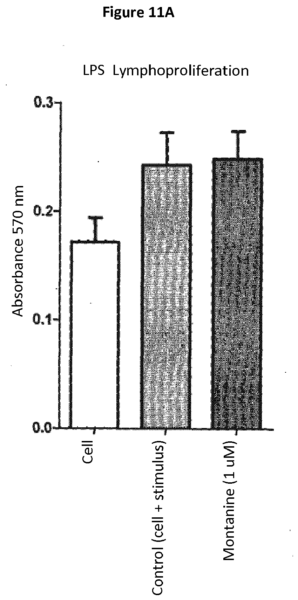

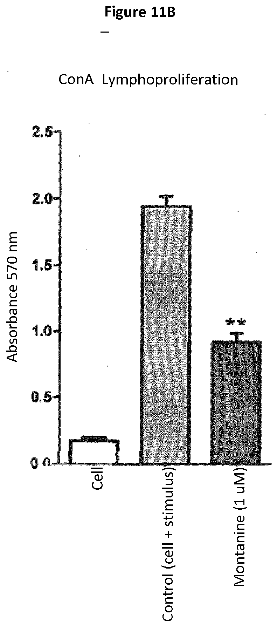

In the performed experiments (Figure 11), montanine had no effect on lymphocyte proliferation stimulated by LPS, but significantly decreased lymphocyte proliferation stimulated by ConA. From these data it can be concluded that montanine has preferential activity on T lymphocytes.

### Synovial fibroblast invasion test

To evaluate the invasion of synovial fibroblasts into matrigel inserts (collagen matrix) a BD kit (Franklin Lakes, NJ, USA) was used, and the test was performed in accordance with the manufacturer specifications.

When cells reached 70-80% confluence, they were trypsinized with trypsin-EDTA for digestion. Then, 2x10⁴ cells were resuspended in 500µl of free culture medium of fetal calf serum and placed on top of the insert. Montanine at a dose of 1 µM or the same concentration of vehicle (DMSO) was added on top of the insert with the cells. At the lower compartment 750µl of culture medium with 10% fetal bovine serum was added. The plate was incubated at 37 °C for 24 h in a stove with 5% CO₂. After the incubation period, the top of the chamber was cleaned with a swab, stained with crystal violet dye, and the total number of cells that invaded the Matrigel membrane was counted in an optical microscope at 100x magnification. Experiments were performed in duplicate.

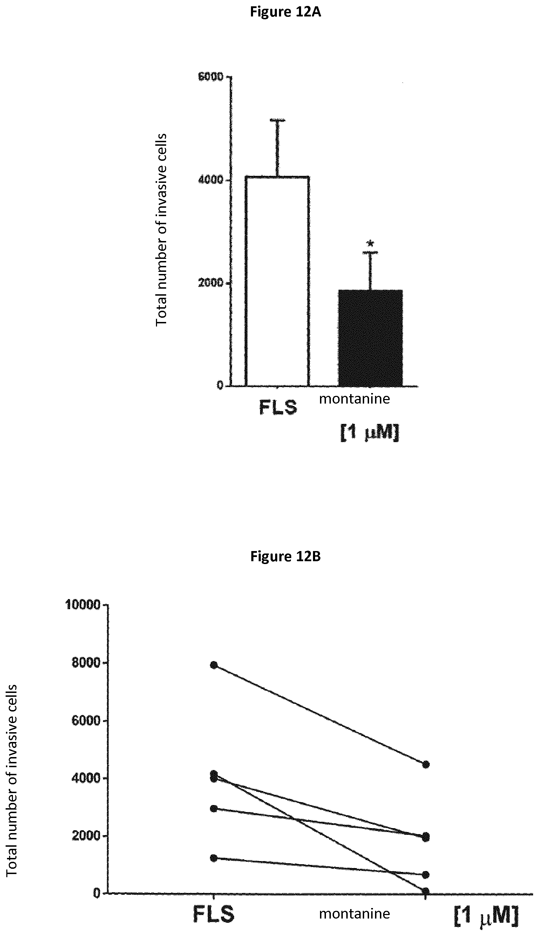

This procedure allows one to compare the normal condition of cell migration and the effect of drugs on this ability. The obtained results demonstrated that montanine reduced the invasion of synovial fibroblasts of the five lines tested (Figures 12A and 12B).

These *in vivo* and *in vitro* results demonstrated the potential of montanine as a potential anti-inflammatory drug. The property of montanine of being able to reduce fibroblast migration and T-lymphocyte proliferation can be understood as proof of concept that montanine is able to modify the disease. Specifically when treating rheumatoid arthritis, a class of drugs can be mentioned, which is known as *disease-modifying antirheumatic drug* (DMARDs), which has the ability to prevent disease progression (from inflammatory to deformant) and not only to treat their symptoms (the particular case of montanine). Montanine, however, can be used as an active capable of modifying the disease when applied to the osteoarticular system diseases. Probably the aforementioned mechanisms of action also act on the treatment and prevention of other diseases having inflammation and/or increased number of fibroblasts in a localized manner as etiopathogenesis, in particular inflammatory and fibrotic lung and kidney diseases, Castleman's disease, psoriatic arthritis and juvenile rheumatoid arthritis. The use of montanine to inhibit fibroblast migration can be associated with intra-articular diseases as well as diseases involving fibroblast dysfunction as a cause or effect, through migration thereof and/or exacerbated or ectopic production of matrix, including fibrosis of organs such as the lungs and kidney.

### References

1. Grespan R, Fukada SY, Lemos HP, Vieira SM, Napimoga MH, Teixeira MM, Fraser AR, Liew FY, McInnes IB, Cunha FQ. CXCR2-specific chemokines mediate leukotriene B4-dependent recruitment of neutrophils to inflamed joints in mice with antigen-induced arthritis. Arthritis Rheum. 200; 58(7):2030-40. 5
2. Tomita T, Kakiuchi Y, Tsao PS. THR0921, a novel peroxisome proliferator-activated receptor gamma agonist, reduces the severity of collagen-induced arthritis. Arthritis Res Ther. 2006;8(1):R7.
3. Pinto LG, Cunha TM, Vieira SM, Lemos HP, Verri WA Jr, Cunha FQ, Ferreira SH. IL-17 mediates articular hypernociception in antigen-induced arthritis in mice. Pain. 2010;148(2)-.247-56.
4. Oliveira PG, Grespan R, Pinto LG, Meurer L, Brenol JC, Roesler R, Schwartsmann G, Cunha FQ, Xavier RM. Protective effect of RC-3095, an antagonist of the gastrin-releasing peptide receptor, in experimental arthritis. Arthritis Rheum. 2011; 63(10):2956-65.

## Claims

1. An alkaloid fraction of *Bhodophiala Bifida* (Herb.) Traub for use in the treatment or prevention of inflammation, rheumatoid arthritis, ulcerative colitis, sepsis, acute pulmonary disease, inflammatory infections, such as inflammatory and/or fibrotic lung and/or kidney diseases, osteoporosis, Castleman's disease, psoriatic arthritis, juvenile rheumatoid arthritis or further non-specific inflammatory joint diseases.

2. A pharmaceutically acceptable composition comprising an alkaloid fraction for use as defined in claim 1.

3. Montanine for use in the treatment or prevention of diseases having migration - invasion or ectopic proliferation of fibroblasts as etiopathogenesis, such as rheumatoid arthritis and juvenile rheumatoid arthritis; or for use in the treatment or prevention of diseases whose etiopathogenesis derives from the inflammatory process, lymphocyte migration and/or proliferation, such as non-specific acute and chronic arthritis, psoriatic arthritis and Castleman's disease; or for use in preventing or treating inflammatory and/or fibrotic diseases of the osteo-articular system, particularly osteoporosis, fibrotic lung forms of rheumatoid arthritis, idiopathic pulmonary fibrosis or renal or retroperitoneal fibrosis; or for use to modify the course of disease without causing an impact or depressing the immune system.

4. A pharmaceutically acceptable composition comprising montanine for use as defined in claim 3.
